# EUROPEAN PATENT APPLICATION

(11) **EP 2 430 975 A1**
(43) Date of publication of application: **21.03.2012**
(21) Application number: 11181173.3
(22) Date of filing: 14.09.2011
(51) Int. Cl.: A61B 5/0452

(54) **Principal component analysis or independent component analysis applied to ambulatory electrocardiogram signals**

(30) Priority: 17.09.2010 US 384012 P; 23.12.2010 US 426686 P
(71) Applicant: Stichting IMEC Nederland, 5656 AE Eindhoven (NL)
(72) Inventor: Romero, Inaki, 5611 PC Eindhoven (NL)
(74) Representative: Mackett, Margaret Dawn

(57) **Abstract**

Described herein is a method and system for adaptively processing ambulatory electrocardiogram (ECG) signals to reduce noise in the output signals. Principal component analysis (PCA) or independent component analysis (ICA) is used to transform multiple input ECG signals into principal or independent components in component space in accordance with reference signals. The transformation is dynamically adjusted in accordance with noise characteristics within the ECG signals. A subset of principal or independent components is selected in accordance with the noise characteristics determined from the ECG signals, from additional non-ECG related signals and from environment- related signals. The subset is processed in accordance with the noise characteristics. In one embodiment, an inverse transform is applied to transform the principal or independent components back to ECG signals with reduced noise from which ECG properties can more accurately be determined. In another embodiment, the selected subset of principal or independent components may have a beat detection or arrhythmia detection algorithm applied directly thereto to determine instantaneous heart rate values or atrial activity.

## Description

### Field of the Invention

The present invention relates to adaptive processing of ambulatory electrocardiogram (ECG) signals and is more particularly, although not exclusively, concerned with reducing the presence of noise in such ECG signals.

### Background to the Invention

Ambulatory monitoring of ECG signals is a diagnostic technique that is widely used by cardiologists. However, these recordings are often affected by high levels of noise due to several sources of noise such as motion artifacts. Motion artifact is the noise introduced to a biopotential signal that results from motion of the measurement electrode. The electrode movement causes deformation of the skin around it making changes in the electrical characteristics of the skin. These electrical changes are then registered in the recorded signal as a motion artifact. Motion artifacts with high amplitude produce a significant reduction in the quality of an ECG signal.

Several methods for noise reduction and motion artifact removal are known in the art. Stress ECG enhancement algorithms are discussed in "Comparing Stress ECG Enhancement Algorithms" by Afonso V, Tompkins W, Nguyen T, Michler K, Luo S., IEEE Eng Med Biol Mag 1996;15: pages 37 to 44. The use of adaptive modelling in the time-frequency domain has been discussed by Augustyniak P. in "Separating Cardiac and Muscular ECG Components using Adaptive Modelling in Time-Frequency Domain", Proc. of the WACBE World Congress on Bioengineering 2007.

Traditional de-noising techniques are based on filtering, and adaptive filtering approaches have been proposed which are based on an adequate reference signal, such as, measurement of skin-electrode impedance as described in "Detection Electrode Motion Noise in ECG Signals by Monitoring Electrode Impedance" by Devlin PH, Mark RG, Ketchum JW., Computers in Cardiology 1984; pages 51 to 56, and in "Comparison of Methods for Adaptive Removal of Motion Artefact" by Hamilton P, Curley M, Aimi R, Sae-Hau C., Computers in Cardiology 2000; 27: pages 383 to 386. Skin stretching measured optical sensors are also described in the latter article as well as in "Effect of Adaptive Motion-Artefact Reduction on QRS Detection" by Hamilton P, Curley M, Aimi R. Biomed Instrum Technol 2000; 34: 197 to 202. The use of accelerometers is described in "Adaptive Reduction of Motion Artifact in the Electrocardiogram" by Tong D, Bartels K, Honeyager K.. Proc. Second Joint EMBS/BMES Conf 2002; 2: pages 1403 to 1404, and "Adaptive Noise Cancelling of Motion Artefact in Stress ECG Signals Using Accelerometer" by Raya M, Sison L.. Proc. Second Joint EMBS/BMES Conf 2002; 2: pages 1756 to 1757.

As sources of ECG signals and motion artifacts are uncorrelated, blind source separation (BSS) techniques could be used for separating both signals as described by Hyvärinen A, Oja E. in "Independent Component Analysis: Algorithms and Applications", Neural Networks 2000; 13: pages 411 to 430, and by Castells F, Cebrián A, Millet J. in "The Role of Independent Component Analysis in the Signal Processing of ECG Recordings", Biomed Tech (Berl) 2007; 52(1): pages 18 to 24. However, in order to apply these methods, a multi-lead ECG recording is required in which the different recorded leads should be linearly independent.

BSS provides separation of a set of mixed signals generated by different sources without prior knowledge of the sources or the mixing process. Examples of BSS include Principal Component Analysis (PCA) and Independent Component Analysis (ICA).

PCA has been used for reducing noise in single lead ECG signals segmented in time intervals as described in "Uni-channel PCA for Noise Reduction from ECG Signals" by Palaniappan R, Khoon TE., Proc 1 st International Bioengineering Conference 2004; pages 436 to 439.

PCA is a mathematical process that uses an orthogonal transformation to convert a data set into a set of uncorrelated principal components. The number of principal components is no more than the number of elements in the original data set. The transformation provides a first principal component having the highest variance, with each subsequent principal component having the next highest variance possible provided it is orthogonal and uncorrelated with the preceding principal components. PCA is sensitive to the relative scaling of the original data set.

In addition to PCA, ICA has been used for processing ECG signals for de-noising, data compression, atrial activity extraction and foetal ECG determination. ICA is another computational method of separating a multivariate signal into additive sub-components and minimises mutual information in the signal which maximises the independence between random variables in the multivariate signal. However, these techniques require a method for automatic component selection in order to be used in a stand-alone system as described in "The Role of Independent Component Analysis in the Signal Processing of ECG Recordings" discussed above, and by Castells F, Laguna P, Sornmo L, Bollmann A, Millet-Roig J. in "Principal Component Analysis in ECG Signal Processing", EURASIP J. Appl. Signal Process 2007; 2007(1): pages 98 to 119. Comparative analysis of PCA/ICA for ECG signals is discussed in "A Comparative Analysis of Principal Component and Independent Component Techniques for Electrocardiograms" by Chawla MPS., Neural Comput & Applic 2009; 18: pages 539 to 559.

Reduction of noise and motion artifacts in ambulatory ECG signals is not trivial, particularly in ambulatory ECG signals that may suffer significant changes in noise levels with respect to time.

### Summary of the Invention

It is therefore an object of the present invention to provide a method of improving ECG signal quality by the reduction of the effects of noise and/or motion artifacts.

It is a further object of the present invention to provide a method of using adaptive blind source separation techniques, in particular, PCA and/or ICA to reduce noise in ECG signals to improve beat detection and arrhythmia detection.

In accordance with a first aspect of the present invention, there is provided a method of processing a plurality of ECG signals to identify at least one property thereof, the method comprising the steps of:
a) acquiring a plurality of ECG signals;
b) transforming the acquired ECG signals to a component space to provide a set of components, the components being a representation of the measured ECG signals in the component space;
c) evaluating the set of components;
d) adaptively selecting a subset of components from within the set of components; and
e) processing the selected subset of components to identify said at least one property.

The plurality of ECG signals can be obtained from a multi-channel ECG system or from individual ECG signals obtained from different sources.

The present invention enables the noise in ECG signals to be substantially reduced so that further ECG analysis, for example, the automatic detection of heart beat and/or arrhythmia, is improved. In one particular embodiment, heart beat detection in noisy ECG signals can be performed with increased accuracy using the method of the present invention.

Preferably, step b) comprises using a blind source separation technique. In one embodiment, the blind source separation technique comprises principal component analysis. In another embodiment, the blind source separation technique comprises independent component analysis.

Regardless of whether principal or independent component analysis is used, component parameters are selected in accordance with a time window.

Preferably, step d) comprises determining quality information of the measured plurality of ECG signals and using the determined quality information to select the subset of components. The quality of ECG signals can be determined by looking for features, such as, RMS, statistical parameters (including kurtosis, variance, entropy etc.) or frequency features (including spectral concentration etc.).

In one embodiment, the selection of the subset can be performed automatically without manual intervention.

Ideally, step d) comprises using non-ECG related reference signals to select the subset of components. Examples of non-ECG related reference signals include motion signals that can be determined by using one or more of: an accelerometer signal, an electrode-tissue impedance measurement, a contact impedance measurement, a temperature measurement, and the output from optical and/or stretch sensors. In particular, electrode-tissue impedance or contact impedance provides an estimation of the noise level at the recording electrodes. Optical sensors, such as, optical or stretch sensors can also be used. Other sensors, such as, microphones and temperature sensors, can also be used.

In one embodiment, said at least one property comprises an instantaneous heart rate value, the method further comprising the step of applying a beat detection algorithm to a selected component within the selected subset of components to provide the instantaneous heart rate value.

This allows the retrieval of beat detection information with increased accuracy.

In another embodiment, said at least property comprises atrial activity, the method further comprising the step of detecting atrial fibrillation within a selected component within the selected subset of components to provide the atrial activity.

The method of using adaptive PCA/ICA can be used to isolate atrial activity by selecting a component in component space that carries information relating to atrial fibrillation and processing that selected component to obtain the atrial activity. Here, the information may be de-noised as part of the processing.

Preferably, the method may further comprise the step of applying an inverse transform to the set of components to remove noise from the plurality of ECG signals.

In a further embodiment, the method requires that an inverse transform be applied to the components to obtain de-noised ECG signals.

In accordance with another aspect of the present invention, there is provided a system for processing a plurality of ECG signals to identify at least one property thereof, the system comprising:- measuring means for measuring a plurality of ECG signals; signal processing means for processing the plurality of measured ECG signals as described; and output means for outputting the processed signals.

In addition, the system also performs noise reduction in ambulatory ECG signals. Such processed signals have been de-noised and can be used, for example, for beat detection and/or arrhythmia detection using the ECG signal, with increased accuracy.

The system may also comprise a communication link for transmitting the acquired ECG signals to a receiver unit. The communication link may be wired or wireless. In one embodiment, the ECG signals may be transmitted to the receiver unit and/or a base station where they are processed. In another embodiment, the ECG signals may be processed at the location of the measurement and are then transmitted wirelessly to the receiver unit and/or the base station.

In a further embodiment, the output means may comprise a display for displaying the processed signals.

### Brief Description of the Drawings

For a better understanding of the present invention, reference will now be made, by way of example only, to the accompanying drawings in which:-
Figure 1 illustrates ECG signals with noise and/or motion artifacts;
Figure 2 illustrates a graph of beat detection accuracy against signal-to-noise (SNR);
Figure 3 illustrates a flowchart of a beat detection method in accordance with the present invention;
Figure 4 illustrates the effect of noise on an ECG signal;
Figure 5 illustrates the principle of using PCA on ECG signals in accordance with the present invention;
Figure 6 illustrates a subset of principal components as a function of the SNR;
Figure 7 illustrates a graph of correlation coefficients against SNR;
Figure 8 illustrates SNR improvement against SNR for PCA output;
Figure 9 illustrates the correlation coefficient of the PCA against SNR value;
Figure 10 illustrates a graph showing the effect of applying adaptive PCA or ICA in a comparison with no filtering with respect to correlation coefficient against SNR;
Figure 11 illustrates SNR improvement in relation to SNR for optimised PCA and ICA;
Figure 12 illustrates adaptive PCA or ICA in accordance with the present invention;
Figure 13 illustrates de-noising of ECG signals in accordance with the present invention;
Figure 14 illustrates SNR improvement in relation to SNR for PCA and ICA;
Figure 15 illustrates adaptive PCA or ICA for beat detection in accordance with the present invention;
Figure 16 illustrates adaptive PCA or ICA for atrial activity in accordance with the present invention; and
Figure 17 illustrates adaptive PCA or ICA to identify different types of ECG activity.

### Description of the Invention

The present invention will be described with respect to particular embodiments and with reference to certain drawings but the invention is not limited thereto. The drawings described are only schematic and are non-limiting. In the drawings, the size of some of the elements may be exaggerated and not drawn on scale for illustrative purposes.

It will be understood that the terms "vertical" and "horizontal" are used herein refer to particular orientations of the Figures and these terms are not limitations to the specific embodiments described herein.

Furthermore, the terms "first", "second", "third" and the like in the description, are used for distinguishing between similar elements and not necessarily for describing a sequential or chronological order. The terms are interchangeable under appropriate circumstances and the embodiments of the invention can operate in other sequences than described or illustrated herein.

Moreover, the terms "top", "bottom", "over", "under" and the like in the description and the claims are used for descriptive purposes and not necessarily for describing relative positions. The terms so used are interchangeable under appropriate circumstances and the embodiments of the invention described herein can operate in other orientations than described or illustrated herein.

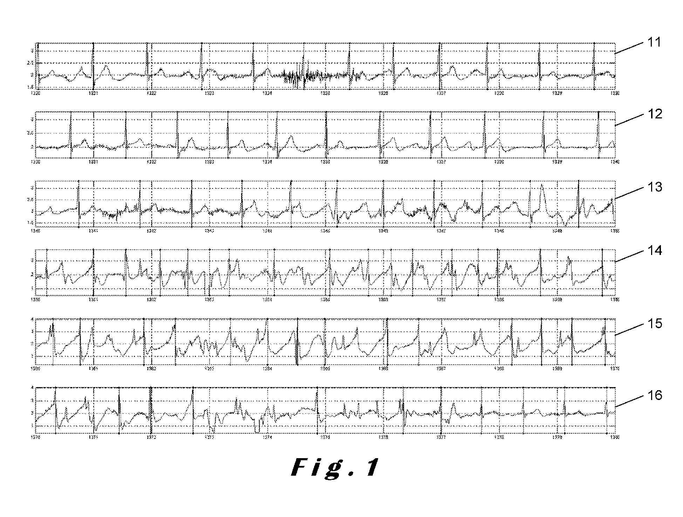

Figure 1 illustrates a plurality of acquired ECG signals with motion artifacts having high amplitude. Six measured ECG signals 11, 12, 13, 14, 15, 16 are shown. In ECG signals 11, 12, 13, the level of noise is low making interpretation of the signals easier. However, due to the presence of noise and/or motion artifacts, it is more difficult to extract actual ECG signals from the measurement ECG signals 14, 15, 16.

One of the most relevant applications in the automatic analysis of ECG signals is the automatic detection of beats. The importance of accurate beat detection is high as this is usually a first step in the development of algorithms for the analysis of ECG signals. The accurate detection of the beat can be used in many clinical applications. It can form the basis for the analysis of rhythm, the analysis of Heart Rate Variability (HRV), detection of pathologies and/or other advanced analyses. Under optimal recording conditions, these methods give detection rates over 99%. However, beat detection accuracy is significantly reduced when the signal quality is decreased due to the presence of motion artifacts.

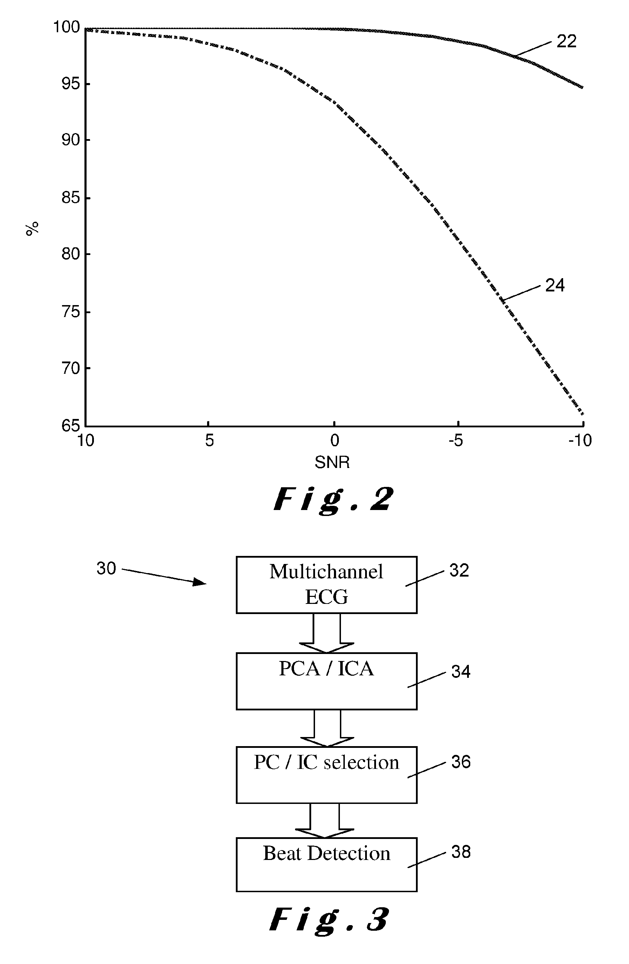

Figure 2 illustrates a graph of detection rate percentage against SNR and shows how positive predictivity, indicated by solid line 22, of a beat detection algorithm decreases rapidly when the value of SNR drops. The sensitivity is also shown as indicated by dotted line 24.

There is still a need to find a method and system which can reduce noise and motion artifacts in ambulatory ECG signals.

According to one embodiment, the present invention relates to a method for performing heart beat detection in noisy ECG signals with increased accuracy. The method comprises acquiring a plurality of ECG signals. These signals may be multi-channel or multi-lead ECG signals or signals obtained from multiple sources. These acquired channels are transformed into signals in a component space by means of PCA or ICA.

As described above, PCA and ICA are techniques which are commonly used in multivariate statistical analysis. The goal of these techniques is the reduction of the number of dimensions from a numerical measurement of several variables for further processing. With this dimensional reduction, PCA and/or ICA can simplify a statistical problem with the minimal lost of information. These methods are also used in signal processing for separating a linear combination of signals generated from sources that are statistically independent. This is achieved by representing the data in a new coordinate system. These transformations are bidirectional and an inverse transformation is carried out to provide the data in the original coordinate system. This is described by Jolliffe IT, in "Principal Component Analysis", Springer Series in Statistics 2002, New York: Springer.

Applying PCA or ICA to n-channel ECG signals that are statistically independent gives n new signals or components. The components corresponding to noise to be cancelled are set to zero and the transformation is inverted to obtain the "filtered" signals in the original coordinate space. It is to be noted that the first new signal or component does not necessarily correspond to the first measured ECG signal. Identifying which components correspond to noise and which correspond to ECG signals is not trivial. Variance and kurtosis can be used for automatic selection of the components.

Variance is a descriptor of a probability distribution in that it provides information about how far variables in a set are spread out from a mean or expected value. Kurtosis is a measure of "peakedness" of a probabilistic distribution of a real-valued random variable. High distributions have sharper peaks and flatter tails, and, low distributions have more rounded peaks and shorter thinner tails.

In Figure 3, a flow chart 30 of the steps involved in beat detection is shown. Multi-channel ECG signals are measured or otherwise acquired in step 32. In addition, non-ECG signals, for example, accelerometer signals, electrode-skin impedance measurements, optical measurements etc,. are preferably also measured or acquired in step 32. The acquired ECG signals are transformed into a set of either principal or independent components, step 34, depending on whether PCA or ICA is to be performed on the measured ECG signals. The principal or independent components are representations of the ECG signals in the component space. From this set of components, a subset is automatically selected, step 36, in accordance with parameters determined from the non-ECG signals also acquired in step 32. The subset is selected based on the determination of the quality of the measured or acquired ECG signals as described with reference to step 32 above. The quality of the ECG signals can be determined from one or more of the non-ECG signals, for example, signals obtained from microphones, light sensors, temperature sensors, gas sensors, humidity sensors or cameras. In one embodiment, by applying a beat detection algorithm, step 38, to this subset of components, improvement in performance is obtained.

In the case of PCA, for high SNR values, retaining the principal components of highest variances give the best performance. When SNR is decreased, the principal components corresponding to highest variance are related to high amplitude noise. In accordance with the present invention, a method for identifying the optimal subset of principal components as a function of input SNR and number of channels is described below. It will be appreciated that a similar process is carried out when using ICA instead of PCA.

In addition to component selection, the time window used to apply PCA or ICA can also be chosen according to noise characteristics of the signals being analysed. The length of the time window for this selection can be adjusted according to the duration of noise in the ECG signals, that is, the shorter the duration of noise, the shorter the time window and the longer the duration of noise, the longer the time window.

In accordance with the present invention, parameters used in ECG signal analysis in ambulatory recordings, such as, the set of components and the time window, are adapted in real-time either independently or in combination.

Experimentation has shown that this adaptation can be carried out based on characteristics of noise in the signal, for example, the length, the level, and frequency bands properties of noise. More generally, this adaptive PCA or ICA method can be based on other parameters that characterise the context of the monitoring environment (called context-aware adaptive PCA or ICA). These parameters may be obtained from the original input multi-channel ECG signals as well as signals from other sources, for example, motion measurements using accelerometers, electrode-tissue or contact impedance measurements, heart rate (when known), and/or optical sensors.

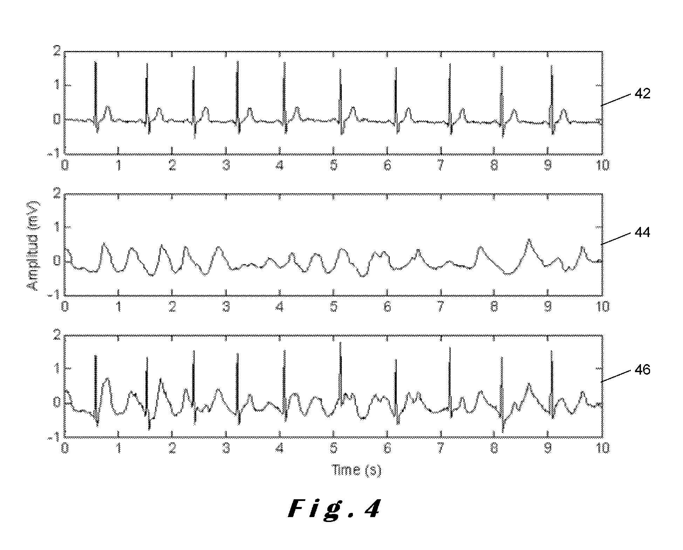

The performance of the PCA and ICA is described below. Clean ECG signals were obtained by recording 8-channel ECG signals, and 8-channel noise only recordings were also obtained. Each 8-channel noise signal was multiplied for a gain factor and added to each 8-channel clean ECG in order to obtain a specific SNR. SNR values ranging from 10 to -10 dB were considered. In Figure 4, an example of a clean ECG signal is shown by trace 42, a pure noise signal is shown by trace 44, and a combination of both signals is shown by trace 46.

PCA was applied to the combined signal 46 to provide principal components, and a subset of principal components was selected in accordance with descending order of variance. The subset of principal components was then inverted, that is, the PCA transform was reversed, to effect filtering out of noise. Similarly, ICA was also applied to the combined signal 46 to provide independent components, and a subset of independent components was selected by kurtosis over a fixed threshold. The subset of independent components was then inverted to effect filtering out of noise.

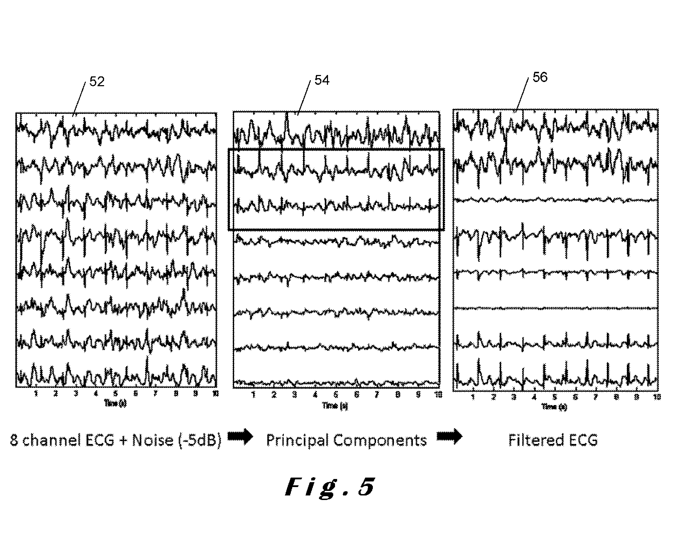

Figure 5 illustrates the application of the invention. An eight-channel ECG signal together with noise at -5dB is shown at 52. Initially, PCA was applied to all 8-channel ECG lead signals at 54. Only one of the 8 resulting principal components was retained and the PCA transform was inverted to obtain the filtered original 8 ECG leads as shown at 56. It can readily be seen that the filtered ECG signals 56 have less noise than the measured ECG signals 52.

Selecting the principal component with highest variance gave in general highest correlation coefficients for high SNR values, that is, over 0dB. However, for SNR values between 0 and -7dB, the principal component which had highest ECG content was the second one with highest variance. Between -8 and -10dB, the principal component which gave the best the highest correlation coefficient was the 4^{th} one.

The optimal number of principal components in function of the SNR was then investigated. PCA was applied to the 8-channel ECG signals. Then, the principal components were sorted by their ranking obtained in descending order. Finally, n components were selected, where n = 1, 2, ..., 8, and the PCA transform was inverted. Overall, the best values were obtained with n = 3 giving a small correlation coefficient improvement with median of 0.02 (MAD = 0.05) and the SNR improvement was in median of 1.49 dB (MAD = 4.62). For low SNR values, PCA performed better when retaining more principal components, for example, 4 principal components for a SNR value of -8dB and 6 principal components for a SNR value between -9dB and -10dB.

Combining the results, the best subset of principal components was selected for each SNR value, for example, principal component 1 corresponds to the value with highest variance, principal component 2 corresponds to the value with the next highest variance, and principal component 8 corresponds to the value with lowest variance.

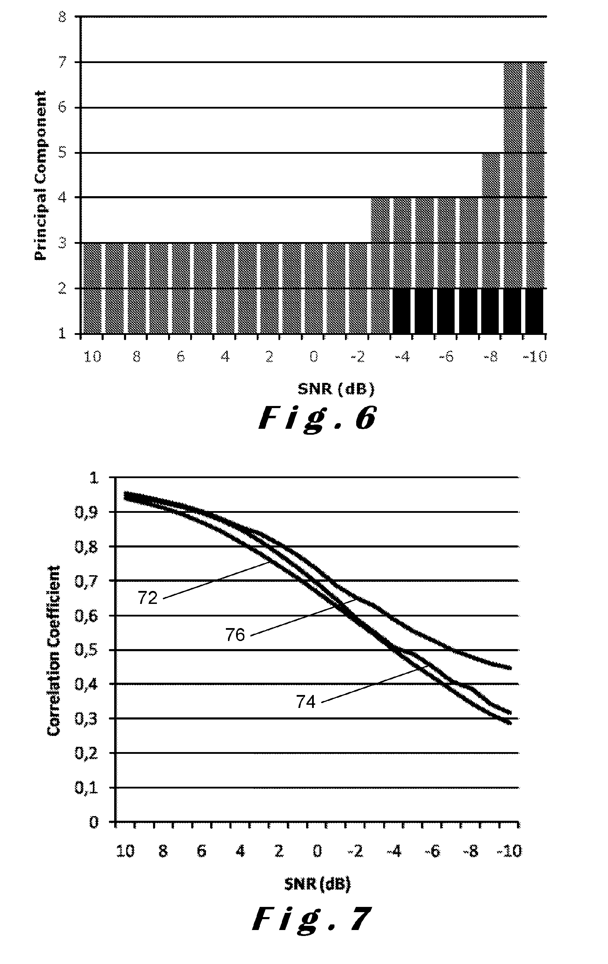

Figure 6 illustrates the subset of principal components that gave best results when considering correlation. It can be seen that for a SNR value between 10dB and -2dB, components 1 to 3 are chosen as the subset. As the SNR increases, different components are chosen. As shown in Figure 6, components 1 to 4 are chosen when the SNR is -3dB, components 2 to 4 are chosen when the SNR is between -4dB and -7dB, components 2 to 5 are chosen when the SNR is between -5dB, and finally components 2 to 7 are chosen when the SNR is between -9dB and -10dB. It will be appreciated that these values are given by way of example only and that any other combination of adjacent components can be chosen in accordance with the SNR values.

The use of this method of component selection was evaluated by comparing its performance with the direct comparison of the noisy ECG signals and the clean ECG signals, that is, with no de-noising, and the use of PCA when the optimal set of principal components was retained for each signal within the dataset and SNR value independently. The optimal set was defined as the one (from all possible combinations) that gave the highest correlation coefficient between the clean ECG signal and the output of the inverted PCA.

For evaluating the signal improvement for PCA and ICA, the correlation coefficient between the noise-free signal and the output after PCA and ICA filtering was determined. In addition, SNR before and after PCA and ICA filtering was estimated. In evaluating the combined signal 46 using PCA and ICA, the optimal subset of principal and independent components respectively was identified for each signal within the dataset and for the SNR independently. The optimal component subset was retained and the PCA or ICA transformation was inverted to provide a filtered 8-channel signal. The optimal component subset was defined as the one that gave the highest correlation coefficient between the clean ECG signal and the output of the inverted PCA or ICA signal. The output of the filtered signal was compared with the clean ECG signal before adding the noise by calculating the correlation coefficient. Median and median absolute deviation (MAD) values of the eight output signals were considered to be representative values for each signal, and median ± MAD values of the whole data set was considered as being representative for each SNR value. Tables 1 and 2 illustrate respectively the correlation coefficients and SNR improvement respectively for PCA and ICA.

**Table 1**

| **SNR (dB)** | **PCA** | **ICA** |
|---|---|---|
| 10 | 0.96 ± 0.01 | 0.95 ± 0.02 |
| 0 | 0.75 ± 0.07 | 0.79 ± 0.07 |
| -5 | 0.55 ± 0.09 | 0.68 ± 0.10 |

**Table 2**

| **SNR (dB)** | **PCA** | **ICA** |
|---|---|---|
| 10 | 2.03 ± 1.22 | 0.00 ± 0.00 |
| 0 | 0.96 ± 0.90 | 3.64 ± 3.40 |
| -5 | 0.73 ± 0.78 | 5.76 ± 4.36 |

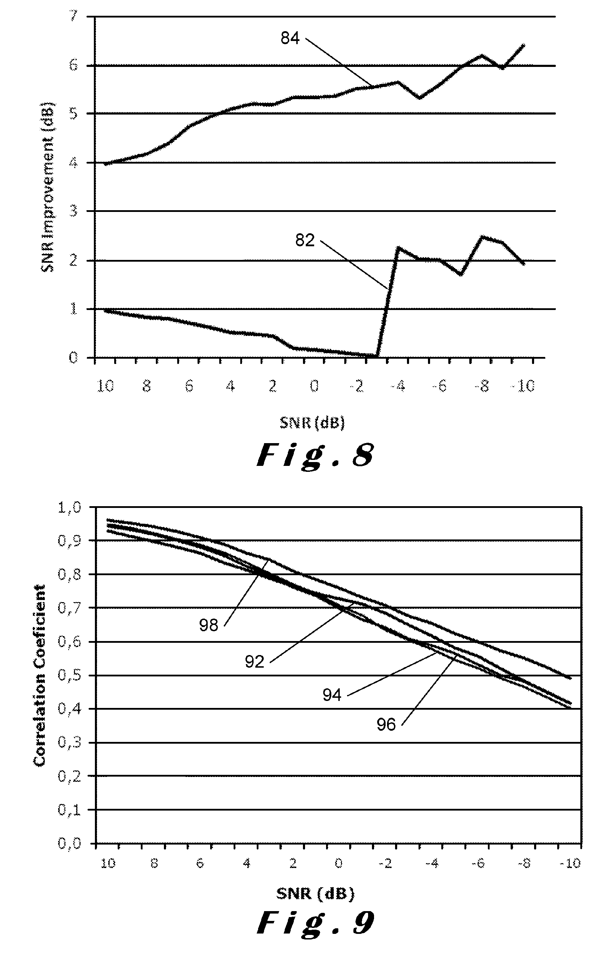

The correlation coefficient results given in Table 1 are illustrated in Figure 7 and the SNR improvement results given in Table 2 are illustrated in Figure 8.

PCA output and clean ECG signals were plotted against SNR value when the selected PC were retained (PCA alg), the optimal subset of principal components were retained (optPCA) and the median correlation coefficient of noisy ECG with clean ECG signals (noPCA). A comparison of the median correlation coefficient for each SNR value is shown in Figure 7 for the case were there is no PCA, line 72, conventional PCA (where the principal components are arbitrarily selected), line 74, and optimised PCA, line 76, in accordance with the invention.

Figure 8 illustrates the median SNR improvement for optimised PCA, line 84, compared to conventional PCA, line 82, for each SNR value. As shown, applying optimised PCA can give a significant improvement, especially with low SNR values, for example, improvement in the correlation coefficient of 0.16 and SNR 6.39dB with SNR = -10dB when the optimal principal components can be indentified for each noisy signal. However, the subset of principal components, as a function of SNR as above, gave a smaller improvement, for example, improvement in the correlation coefficient of 0.03 and SNR 1.92dB with SNR = -10dB.

The effect of the number of input channels was also investigated for both PCA and ICA. In addition to the eight input channels, subsets of input channels of six, four and two channels were also considered. Following the same procedures as described above, for each input subset, the optimal component subset was found for each signal and SNR value. To obtain consistent results, only one lead, common for all subsets, was considered for comparing the input and output of the PCA and ICA.

For PCA, the highest correlation coefficients for high SNR values was obtained for six and eight input channels, for example, at a SNR of 10dB, the correlation coefficient was found to be 0.96 ± 0.02. For SNR values between 1dB and -8dB using two input channels, the highest correlation coefficients were obtained, for example, at a SNR value of 0dB, a correlation coefficient of 0.73 ± 0.10 was obtained. As the SNR decreased, eight input channels gave the highest correlation coefficient of 0.39 ± 0.11 at -10dB.

When ICA was used, higher correlation coefficients were obtained for six and eight input channels over all SNR values. Over 3dB, the correlation coefficient was similar, for example, for a SNR value of 10dB, the correlation coefficient was found to be 0.95 ± 0.02, and for below that value, the difference was small, for example, 0.82 ± 0.07 and 0.84 ± 0.07 respectively at 0dB, and at a SNR value of -10dB, correlation coefficients of 0.43 ± 0.15 and 0.46 ± 0.16 were obtained respectively. In contrast, two input channels provided lower correlation coefficients, for example, 0.94 ± 0.04 and 0.32 ± 0.13 at SNR values of 10dB and -10dB respectively.

In addition, the SNR before and applying PCA or ICA was estimated in order to calculate the SNR improvement. For PCA, using eight input channels, the highest SNR improvement was obtained for SNR values down to 4dB, and between 3dB and 0dB, the highest improvement was obtained using six input channels. Between SNR values of -1dB and -8dB, two input channels provided the best SNR improvement and for lower SNR values, eight input channels provided the best SNR improvement.

ICA based filtering provided higher SNR improvements when using six or eight input channels. Using two input channels, the lowest SNR improvement was obtained for all SNR values. The SNR improvement was plotted against input SNR values for both PCA and ICA as shown in Figure 9.

Figure 9 illustrates the correlation coefficient results for different numbers of input channels against the different values of SNR. Respective lines 92, 94, 96, 98 refer to two, four, six and eight input channels. Decreasing the number of input channels did not yield a big drop in the correlation coefficient, namely, a median drop of 0.49 and 0.42 for eight and two input channels respectively at SNR = -10dB). The SNR improvement dropped in median value from 4.35dB down to 0.38dB (eight and two input channels) at SNR = -10dB.

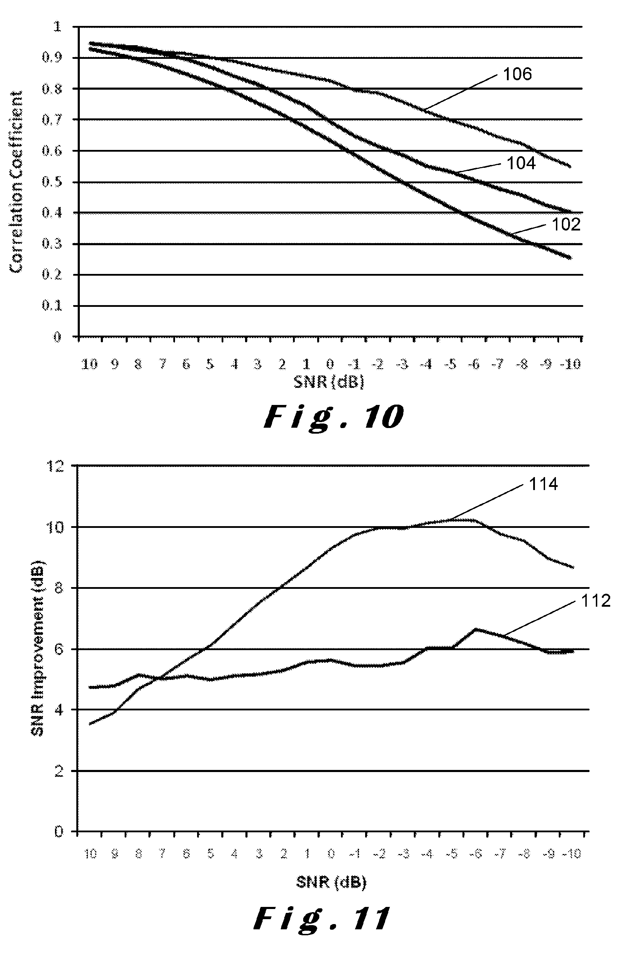

Figures 10 and 11 illustrate respectively improvement in correlation coefficient and SNR improvement. In Figure 10, the difference obtained for the correlation coefficient against SNR when there is no filtering using either PCA or ICA (trace 102), using an optimised PCA (trace 104), and using an optimised ICA (trace 106). Similarly, in Figure 11, the SNR improvement against SNR is shown for an optimised PCA (trace 112) and an optimised ICA (trace 114) respectively.

In addition, the effect of a "fixed" subset of components for each SNR value was investigated. For each value of the input SNR, the components were sorted in descending order by variance in the case of PCA and by kurtosis in the case of ICA. The subset of components that gave the highest median value of the correlation coefficient between filtered and clean ECG signals was identified for each input SBR. These fixed component subsets were used to test an automatic PCA or fixed-PCA and automatic ICA or fixed-ICA.

It was found that the difference in the correlation coefficient, when comparing clean ECG signals with filtered signals, using fixed-PCA led to a significantly lower performance than not applying any filtering fro SNR values of 0dB and above. Below 0dB, fixed-ICA had a higher median correlation coefficient than no filtering. For the SNR improvement determination, fixed-PCA provided an improvement of 3.86 ± 1.59dB at 10dB. The performance was lower when the input SNR decreased, for example, at a SNR value of -10dB, the SNR improvement was determinet to be 1.03 ± 2.56dB. Fixed-ICA provided low improvement in SNR for high input SNR values, for example, at a SNR of 10dB, the improvement was 0.45 ± 2.40dB. However, lower input SNR values provided higher performance, for example, at a SNR value of -5dB, the SNR improvement was found to be 7.50 ± 4.13dB.

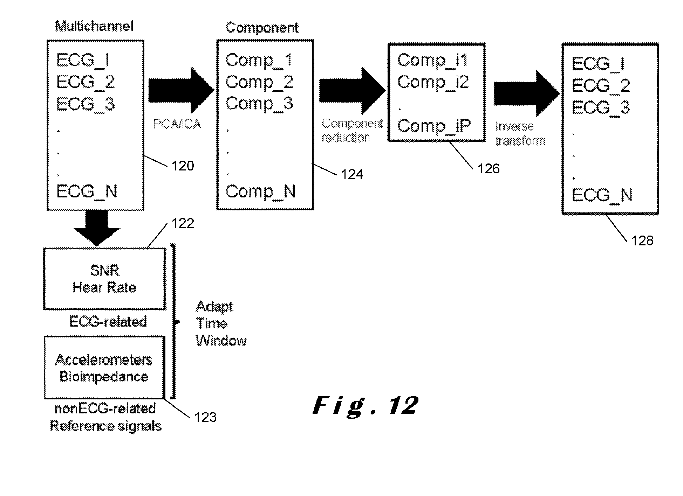

Figure 12 illustrates an implementation of the present invention in which ECG signals are processed to remove the noise. A multi-channel ECG 120, signals ECG_1 to ECG_N, is taken from which SNR and heart rate can be derived to provide an ECG-related input 122. Non-ECG related signals are also taken, that form a non-ECG input 123, which, in this embodiment, are indicative of motion of the subject, and hence motion artifacts. Although only motion sensors are shown, it will be appreciated that other sensors can also be utilised as discussed above.

PCA or ICA is applied to the multi-channel ECG 120 to produce components Comp_1 to Comp_N in component space 124. Using the ECG-related input signal 122 and the non-ECG related input reference signal 123 are used to adapt a time window for the transformation into component space 124. The time window can be considered to be estimated from the time variant properties of the noise that can be estimated from the SNR determined in the input ECG signals or from additional non-ECG signals. From the component space 124, the number of components 126 selected is determined in accordance with the noise properties (such as, SNR and other time-frequency parameters), or according to other parameters derived from the ECG or non-ECG input signals. An inverse transform is applied on the selected components to provide a multi-channel ECG signal 128 with less noise than the measured multi-channel ECG signal 120.

This system can be adapted in accordance with signals determined from the environment and can be dynamically modified, for example, in accordance with the time window and/or component selection, based on changes in the environment. This adaptive approach effectively de-noises the ECG signals to improve the performance of subsequent ECG processing compared to traditional PCA or ICA approaches.

Here, the set of components, when using either PCA or ICA, can be used for reconstruction based on the noise of the signal, or more generally, based on the context of the monitoring environment. Here, reference signals 132 are used in conjunction with the ECG signals 130 to define the components 134 using either PCA or ICA. Component reduction 136 is determined and an inverse transform is applied to provide de-noised ECG signals 138. As discussed above, the selection of the components is determined from the actual ECG signals and the environment, the selection being dynamically modified based on the change in the environment. This is in contrast to current PCA/ICA techniques where the component selection is pre-defined and static, and hence, the component selection is not adaptable to changes in the environment.

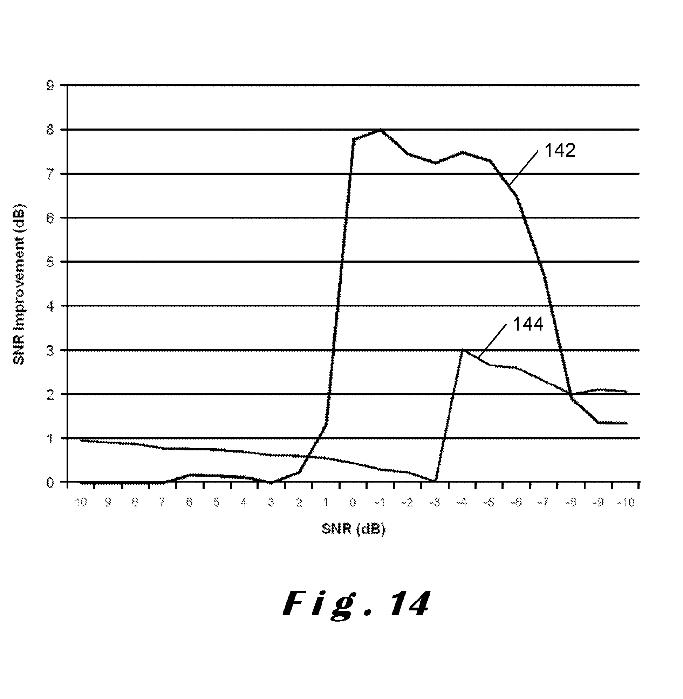

Figure 14 illustrates adaptive PCA/ICA for de-noising in accordance with the invention. Here, ICA is indicated by trace 142 and PCA by trace 144. It is shown that ICA can provide better performance.

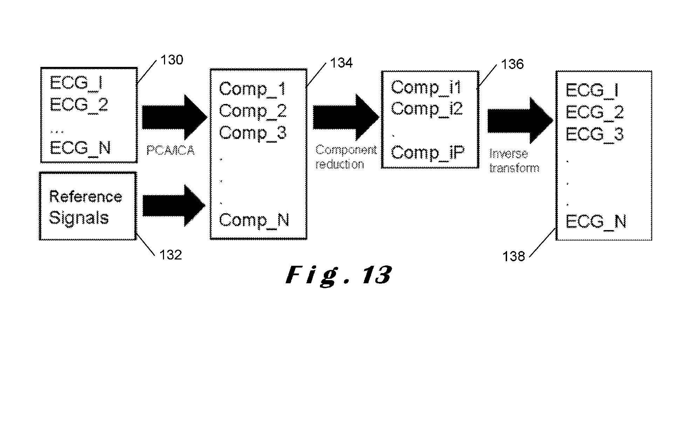

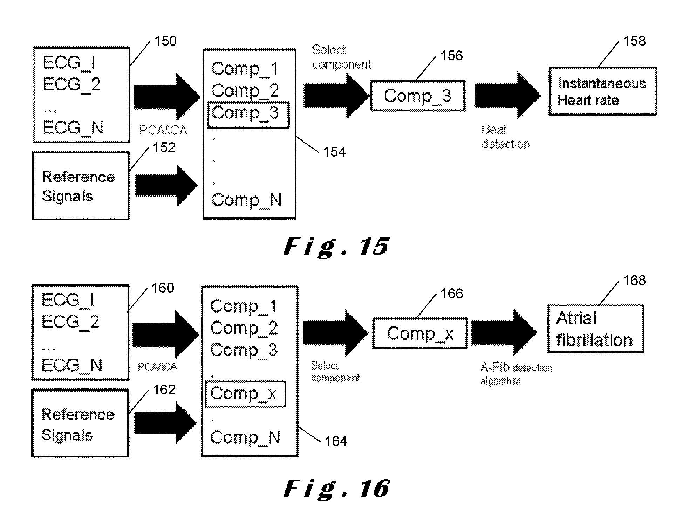

In Figure 15, beat detection can be carried out in component space without having to apply the inverse transform as described with reference to Figures 12 and 13. Here, a multi-channel ECG signal 150 is converted to component space 154 using reference signals 152. A single component 156 is selected and from this component, an instantaneous heart rate value 158 can be determined. This is in contrast to the conventional beat detection where the instantaneous heart rate is determined only once the inverse transform has been applied to the selected component(s). The advantages of determining the instantaneous heart rate or beat detection in the component space include an increased robustness to noise, lower computing complexity, and the component selection is carried out dynamically using adaptive PCA or ICA. This additionally provides a positive detection of beats and does not detect false beats.

A further option for using adaptive PCA or ICA as described above is in atrial activity detection. Here, PCA or ICA is used to extract a specific wave containing information about atrial activity and an optimal component containing this information can be identified in real-time. Figure 16 illustrates the adaptive method in accordance with the invention for atrial activity detection. This is similar to that shown in Figure 15 but with the component that carries almost exclusively the information on the atrial fibrillation wave being selected. In a similar way to Figure 14, the multi-channel input signals 160 are transformed to component space 164 using reference signals 162. A single component 166 is selected in component space and 164 and an atrial fibrillation algorithm applied to determine the presence of atrial fibrillation 168. Again, no inverse transform is required and the relevant atrial activity can be isolated in the component space. This has the advantages of increased sensitivity and automatic component selection due to only one component carrying the information of interest for the detection of atrial activity.

The effect of using PCA and ICA for beat detection was evaluated in comparison to the situation where no filtering was applied for SNR values between 10dB and -10dB, in 1dB steps. Here, the PCA and ICA transformations are inverted before the beat detection algorithm was applied. In each case, an optimal component subset was selected that gave the highest correlation coefficient between a clean ECG signal and the output of the inverted PCA or ICA. Sensitivity and positive predictability were considered for optimal component selection. In each case, a beat detection algorithm was applied to the filtered signals produced by PCA and ICA and to the original signal before filtering.

In terms of sensitivity, the beat detector had a good performance with a sensitivity of 100% down to -6dB. In terms of positive predictability, a value of 100% was obtained down to 6dB and, below 6dB, the value dropped to 83.09% at 0dB and 48.81 % at -10dB. When both PCA and ICA were applied using the optimal component subset, a sensitivity of 100% was obtained for all SNR values in the range.

For positive predictability, PCA yielded an improvement for all SNR values with values of 95.45% at a SNR value of 0dB and 56.87% for a SNR value of -10dB. However, ICA filtering gave a higher performance for positive predictability for all SNR values, for example, 100% for 0dB and 61.38% for -1 0dB.

In accordance with the present invention, an automatic selection of principal and independent components is required without human intervention. Here, automatic component selection was carried out using kurtosis to identify which components correspond to ECG information. Components having a kurtosis over a fixed threshold were selected whilst components below this threshold were rejected. If none of the components had a kurtosis over the fixed threshold, then the component with the highest kurtosis was selected. Again, positive predictability and sensitivity were considered.

For positive predictability, a value of 100% was obtained for SNR values down to 3dB when no filtering was applied. However, below a SNR value of 3dB, the positive predictability dropped considerably to 57.43% when the SNR was -10dB.

Applying PCA and selecting the components automatically gave a higher positive predictability than the case with no filtering for all SNR values below 3dB with values of 100% for all SNR values down to 0dB and 58.82% for a SNR value at -10dB. It was found that PCA with optimal component selection outperformed PCA with automatic selection for all SNR values below 0dB.

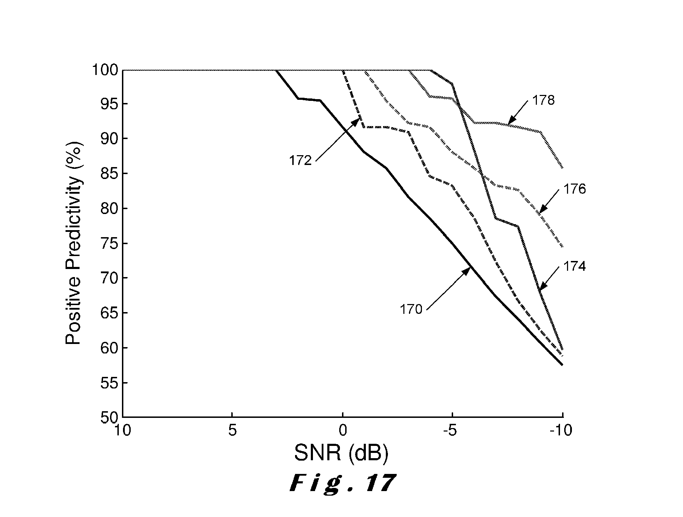

ICA with automatic component selection was found to give a higher positive predictability values when compared to PCA with automatic component selection, for example, down to -4dB a value of 100% was obtained and at -10dB a value of 59.69% was obtained. In addition, ICA with the optimal component subset gave higher positive predictability values than those obtained for both versions of PCA and for ICA with automatic component selection for SNR values below -5dB. The results obtained are shown in Figure 17.

In Figure 17, a graph of positive predictability against SNR value is shown. Line 170 relates to the results obtained with no filtering; line 172 relates to the results obtained for PCA with automatic component selection; line 174 relates to the results obtained for ICA with automatic component selection; line 176 relates to the results obtained for PCA with optimal component selection; and line 178 relates to the results obtained for ICA with optimal component selection.

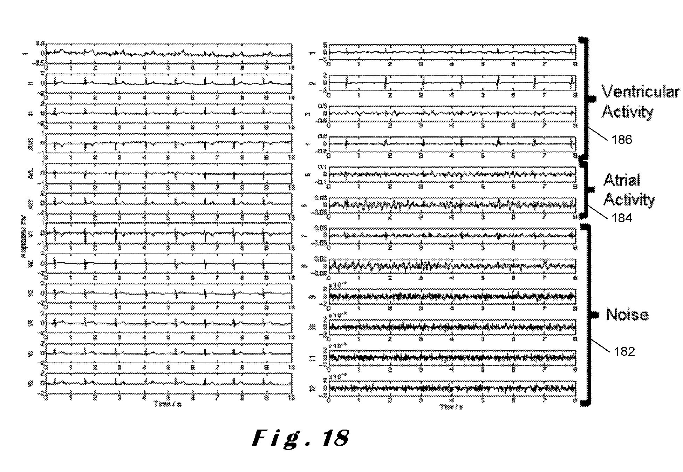

Figure 18 shows a multi-channel ECG that, after adaptive PCA or ICA, can provide information relating to noise 182, atrial activity 184 and ventricular activity 186.

Although PCA and ICA have been described as being alternatives, there may be some applications where both PCA and ICA need to be applied in succession. In addition, a combination of PCA-ICA could also be applied.

While the above detailed description has shown, described, and pointed out novel features of the invention as applied to various embodiments, it will be understood that various omissions, substitutions, and changes in the form and details of the device or process illustrated may be made by those skilled in the technology without departing from the invention.

## Claims

1. A method of processing a plurality of ECG signals (ECG_1, ECG_2, ..., ECG_N) to identify at least one property thereof, the method comprising the steps of:
a) acquiring a plurality of ECG signals (120; 130; 150; 160);
b) transforming the acquired ECG signals (120; 130; 150; 160) to a component space (124, 134, 154, 164) to provide a set of components (Comp_1, Comp_2, Comp_3, ..., Comp_N), the components (Comp_1, Comp_2, Comp_3, ..., Comp_N) being a representation of the measured ECG signals (120; 130; 150; 160) in the component space (124, 134, 154, 164);
c) evaluating the set of components (Comp_1, Comp_2, Comp_3, ..., Comp_N);
d) adaptively selecting a subset (126, 136, 156, 166) of components (Comp_i1, Comp_i2, Comp_i3, ..., Comp_iP) from within the set of components (Comp_1, Comp_2, Comp_3, ..., Comp_N); and
e) processing the selected subset (126, 136, 156, 166) of components (Comp_i1, Comp_i2, Comp_i3, ..., Comp_iP) to identify said at least one property.

2. A method according to claim 1, wherein step b) comprises using a blind source separation technique.

3. A method according to claim 2, wherein the blind source separation technique comprises principal component analsyis.

4. A method according to claim 2 or 3, wherein the blind source separation technique comprises independent component analysis.

5. A method according to claim 3 or 4, further comprising the step of selecting component parameters in accordance with a time window.

6. A method according to any one of the preceding claims, wherein step d) comprises determining quality information of the measured ECG signals (120; 130; 150; 160), and using the determined quality information to select the subset (126, 136, 156, 166) of components (Comp_i1, Comp_i2, Comp_i3, ..., Comp_iP).

7. A method according to any one of the preceding claims, wherein step d) comprises using non-ECG related reference signals (122, 132, 152, 162) to select the subset (126, 136, 156, 166) of components (Comp_i1, Comp_i2, Comp_i3, ..., Comp_iP).

8. A method according to claim 6, wherein the non-ECG related reference signal (122, 132, 152, 162) comprises one or more of:
an accelerometer signal, an electrode-tissue impedance measurement, a contact impedance measurement, a temperature measurement, and the output from optical and/or stretch sensors.

9. A method according to any one of the preceding claims, wherein said at least one property comprises an instantaneous heart rate value (158), the method further comprising the step of applying a beat detection algorithm to a selected component (156) within the selected subset of components to provide the instantaneous heart rate value (158).

10. A method according to any one of claims 1 to 9, wherein said at least property comprises atrial activity (168), the method further comprising the step of detecting atrial fibrillation within a selected component within the selected subset (166) of components to provide the atrial activity.

11. A method according to any one of claims 1 to 8, further comprising the step of applying an inverse transform to the set of components to remove noise from the plurality of ECG signals (128, 138, 158, 168).

12. A system for processing a plurality of ECG signals to identify at least one property thereof, the system comprising:-
measuring means for measuring a plurality of ECG signals; signal processing means for processing the plurality of acquired ECG signals according to any one of the preceding claims; and
output means for outputting the processed signals.

13. A system according to claim 11, further comprising a receiver unit and a communication link for transmitting the acquired ECG signals to the receiver unit.

14. A system according to claim 13, wherein the communication link comprises a wireless communications link.

15. A system according to any one of claim 12 to 14, wherein the output means comprises a display for displaying the processed signals.
